(19) **Europäisches Patentamt**
European Patent Office
Office européen des brevets

(11) **EP 1 688 109 B1**

(12) **EUROPÄISCHE PATENTSCHRIFT**

(45) Veröffentlichungstag und Bekanntmachung des Hinweises auf die Patenterteilung:
**21.07.2010 Patentblatt 2010/29**

(51) Int Cl.:
***A61F 13/00*** *(2006.01)*

(21) Anmeldenummer: **06000893.5**

(22) Anmeldetag: **17.01.2006**

(54) **Wundauflage**

Wound dressing

Pansement

(84) Benannte Vertragsstaaten:
**AT BE BG CH CY CZ DE DK EE ES FI FR GB GR HU IE IS IT LI LT LU LV MC NL PL PT RO SE SI SK TR**

(30) Priorität: **02.02.2005 DE 102005004924**

(43) Veröffentlichungstag der Anmeldung:
**09.08.2006 Patentblatt 2006/32**

(73) Patentinhaber: **Paul Hartmann Aktiengesellschaft 89522 Heidenheim (DE)**

(72) Erfinder: **Wesp, Hansjörg, Dr.
89551 Königsbronn (DE)**

(56) Entgegenhaltungen:
**WO-A-01/05445    US-A1- 2004 260 253**

**Beschreibung**

Die Erfindung betrifft eine Vorrichtung mit einem Mittel zur Erzeugung von Kohlenstoffdioxid zur Behandlung von trophischen Störungen in der Haut und in Wunden.

Unter dem Begriff Trophik wird allgemein der Ernährungszustand eines Gewebes oder Organs verstanden. Trophische Störungen sind demnach ernährungs- und/oder wachstumsbedingte Störungen des Gewebes oder der Organe. Die hierbei auftretenden Beschwerden, wie etwa Stauungsdermatosen oder Ulcus cruris venosum, sind dabei die schwersten Folgen einer chronischen venösen Insuffizienz. Diese werden durch eine konstitutionelle, anlagenbedingte Venenwandschwäche oder als Folge thrombotischer Erkrankungen hervorgerufen. Ebenfalls kann man die Dekubitalulcera zu den trophischen Hautstörungen zählen, wobei man die Dekubitalulceraerkrankungen auf regionale Durchblutungsstörungen, die besonders oft bei immobilisierten bettlägerigen Patienten auftreten, zurückführen kann. Hierbei wird diese Erkrankung noch durch Benetzung der entsprechenden Hautflächen mit Ham oder Schweiß gefördert.

Der Dekubitus ist definiert als durch äußere (längerfristige) Druckeinwirkung mit Kompression von Gefäßen und lokaler Ischämie hervorgerufene trophische Störung von Geweben (v. a. Haut und Unterhautgewebe) mit Nekrose, Mazeration, evtl. Infektion. Dekubita entstehen vor allem bei Bettlägerigkeit, insbesondere an Körperstellen, an denen die Haut dem Knochen unmittelbar anliegt, aber auch z.B. unter schlecht sitzenden Prothesen und zu engen Gipsverbänden.

Der Dekubitus wird in folgende Stadien eingeteilt:

- Persistierende, umschriebene Hautrötung bei intakter Haut; weitere klinische Zeichen können Ödembildung, Verhärtung und eine lokale Überwärmung sein.
- Teilverlust der Haut; Epidermis bis hin zu Anteilen der Dermis sind geschädigt; der Druckschaden ist oberflächlich und kann sich als Blase, Hautabschürfung oder flaches Geschwür darstellen.
- Verlust aller Hautschichten und Schädigung oder Nekrose des subkutanen Gewebes, die bis auf die darunter liegende Faszie reichen kann; der Dekubitus zeigt sich klinisch als tiefes, offenes Geschwür.
- Verlust aller Hautschichten mit ausgedehnter Zerstörung, Gewebsnekrose oder Schädigung von Muskeln, Knochen oder unterstützenden Strukturen (Sehnen, Gelenkkapseln.

Zur Behandlung von trophischen Störungen in der Haut sind im Stand der Technik verschiedenste Methoden bekannt. Die meisten der Methoden greifen auf Pharmaka zurück, die in den verschieden Stadien einer Wundheilung angewendet werden. Als eine Methode neuerer Art steht die Verwendung von Kohlenstoffdioxid als Behandlungsmittel zur Untersuchung. Beim Einsatz von Kohlenstoffdioxid wird das Kohlenstoffdioxid als Trockenbad (Gasbad) oder auch in Form von gashaltigen Thermalwässern zur Behandlung in der physikalischen Therapie eingesetzt.

Kohlenstoffdioxid ($CO_2$) ist ein farbloses, geruchloses und nicht brennbares Gas. Der Ausdruck Kohlendioxid ist als Trivialname gebräuchlich und soll in der vorliegenden Schrift dieselbe Bedeutung haben. Kohlenstoffdioxid entsteht z.B. bei der Verbrennung von kohlenstoffhaltigen Substanzen, wenn Sauerstoff vorhanden ist. Auch im Organismus entsteht Kohlenstoffdioxid als Nebenprodukt der Zellatmung. Das $CO_2$ wird dabei über den Atem abgegeben. Umgekehrt verwenden Pflanzen bei der Photosynthese Kohlenstoffdioxid, um Glukose zu produzieren und ihre Biomasse aufzubauen.

In Wasser gelöstes Kohlenstoffdioxid bildet Kohlensäure $H_2CO_3$, wobei ca. 99% des Kohlenstoffdioxids nur physikalisch gelöst sind. Die Kohlensäure als solche liegt jedoch praktisch nicht als Molekül vor, sondern dissoziiert in $H^+$, $HCO_3^-$ und $CO_3^{2-}$, die je nach Druck, Temperatur und pH-Wert in wechselnden Verhältnissen miteinander im Gleichgewicht stehen. Fängt man Hydronium-Ionen ($H_3O^+$) unter Zugabe von Hydroxidionen ($OH^-$) ab, so löst sich Kohlenstoffdioxid in der Lauge vollständig unter Bildung von Carbonaten. Daher kann der pH-Wert $CO_2$-haltiger Wasserlösungen höchstens auf 3,7 (Normaldruck) bzw. 3,3 (Grenzwert unter Druck) absinken.

Kohlenstoffdioxid findet im festen Aggregatzustand unter der Bezeichnung Trockeneis Anwendung in der Technik. Trockeneis sublimiert bei -78 °C. Bei der Sublimation entsteht ein weißer Nebel aus dem kaltem $CO_2$-Luft-Gemisch und kondensierender Luftfeuchtigkeit, der als Nebel-Effekt in der Bühnentechnik Einsatz findet.

Viele Getränke enthalten Kohlenstoffdioxid. Bei manchen Getränken entsteht $CO_2$ durch Gärung (Bier, Sekt), bei anderen wird es künstlich zugesetzt (Limonade, Sodawasser).

Als Lebensmittelzusatzstoff trägt es die Bezeichnung E 290.

Überkritisches Kohlenstoffdioxid kann in vielen Anwendungsbereichen giftige organische Lösungsmittel ersetzen und wird auch in der Halbleiterindustrie verwendet. Auch zur Extraktion von Naturstoffen wie z. B. Koffein aus Pflanzen wird superkritisches Kohlenstoffdioxid verwendet.

Kohlendioxid und Wasser sind hinsichtlich der Menge die wichtigsten Endprodukte des Stoffwechsels. Im Wasser gelöstes Kohlendioxid reagiert zur zweiprotonigen Kohlensäure (1), welche in einer zweiten Reaktion dissoziiert (2).

$$(1) \qquad CO_2 + H_2O \rightleftarrows H_2CO_3 \qquad pK = 3{,}1$$
$$(2) \qquad H_2CO_2 + H_2O \rightleftarrows HCO_3^- + H_3O^+ \qquad pK_{s1} = 3{,}3$$
$$(3)\ \text{Gesamtreaktion} \qquad CO_2 + 2\,H_2O \rightleftarrows HCO_3^- + H_3O^+ \qquad pK_s = 6{,}4$$

Das Gleichgewicht der ersten Reaktion liegt auf der linken Seite (pK = 3,1). Löst man also Kohlendioxid in Wasser liegt es vorwiegend als $CO_2$ und nur zu einem geringen Teil als Kohlensäure ($H_2CO_3$) vor. Kohlensäure selbst reagiert als schwache Säure. Die konjugierte Base ist das Hydrogenkarbonat, auch Bikarbonat genannt. Das Gleichgewicht der Gesamtreaktion liegt weit auf der Eduktseite.

Kohlendioxid und Bikarbonat bilden ein Puffersystem mit pH-Optimum bei pH = 6,4.

$$(4) \qquad pH = 6{,}4 + \log \frac{[HCO_3^-]}{[CO_2]}$$

Das Kohlendioxid-Bikarbonatpuffersystem ist das bedeutendste Puffersystem des Blutes, das auf einen pH von 7,4 gepuffert ist. Das Konzentrationsverhältnis der Puffersubstanzen bei pH = 7,4 lässt sich somit berechnen:

$$(5) \qquad 10^{(pH-pK_s)} = 10^1 = \frac{[HCO_3^-]}{[CO_2]} = \frac{10}{1}$$

Die Reaktionskonstante ist temperaturabhängig; der $pK_s$ der Gesamtreaktion beträgt im Blut bei 37 °C 6,1 statt 6,4 bei 25 °C. Das Konzentrationsverhältnis wird dann zu:

$$(6) \qquad 10^{(1{,}3)} = \frac{[HCO_3^-]}{[CO_2]} = \frac{20}{1}$$

Es liegt also ein Überschuss an Bikarbonat vor, sodass das Puffersystem vor allem gegen $H_3O^+$-Ionen wirkt. Da $CO_2$ ein Gas ist, steht das gelöste $CO_2$ im Gleichgewicht mit dem $CO_2$ der Luft in der Lunge. Hier zeigt sich eine Besonderheit dieses Puffersystems. Es kann nämlich nicht nur $H_3O^+$-Ionen am Entstehungsort abpuffern, sondern auch das Reaktionsprodukt aus dem Gleichgewicht entfernen:

$$(8) \qquad H_3O^+ + HCO_3^- \rightarrow CO_2 \uparrow H_2O$$

Der senkrechte Pfeil deutet an, dass das Kohlendioxid die Lösung verlässt und in die Gasphase geht (das System verlässt). Man spricht deshalb auch von einem offenen Puffersystem.

Die Chemie des Kohlenstoffdioxids im menschlichen Körper ist unter anderem durch den Bohr Effekt bekannt. Als Bohr Effekt wird die Abhängigkeit des Verlaufs der Sauerstoff-Dissoziationskurve vom pH-Wert und dem Kohlenstoffdioxid-Partialdruck des Blutes bezeichnet. Sinkt der pH-Wert oder steigt der Kohlenstoffdioxid-Partialdruck nimmt die Affinität des Sauerstoffs zum Hämoglobin ab und umgekehrt. Der Bohr-Effekt erleichtert die Sauerstoffbindung im Lungenkreislauf und die Sauerstoffabgabe im Gewebe. D.h., dass unter erhöhtem $CO_2$ Gewebsdruck die Abgabe von Sauerstoff aus dem Hämoglobin steigt und damit mehr Sauerstoff in die Gewebe diffundiert. Hierbei wird die Trophik entscheidend verbessert.

In der Patentliteratur sind darüber hinaus mit der WO 01/ 005 445 A1 Wundauflagen bekannt, die Kohlenstoffdioxid zur Behandlung von geschädigter Haut bereitstellt. Diese. Wundauflage besteht aus einer das Kohlenstoffdioxid generierenden Schicht, einer Wundkontaktschicht, die einen klebenden Teil und ein absorbierendes Pad aufweist. Ein Nachteil dieser Wundauflage besteht darin, dass eine absorbierende Schicht direkt über einer Wunde zu liegen kommt und dadurch keine ausreichende Versorgung der Wunde mit Kohlenstoffdioxid gewährleistet ist. Darüber hinaus kann die Wunde mit einer dieser Schrift entsprechenden Wundauflage nicht ausreichend mit einer Kohlenstoffdioxid enthaltenden wässrigen Lösung umspült werden.

Weiterhin ist mit der US 2004/0260253 A1 eine Wundauflage beschrieben, mittels der ein oder mehrere Gase, wie z.B. Sauerstoff, Stickstoff oder Kohlenstoffdioxid einer Wunde verabreicht werden kann.

Ausgehend vom Stand der Technik liegt eine Aufgabe der vorliegenden Erfindung darin, eine verbesserte Vorrichtung bereitzustellen, die in der Therapie mit Kohlenstoffdioxid zur Behandlung von trophischen Störungen Verwendung finden kann. Eine weitere Aufgabe der Erfindung besteht darin, eine Vorrichtung bereitzustellen, die Kohlenstoffdioxid in der feuchten Wundbehandlung zum Einsatz bringt und die gewährleistet, dass eine ausreichende Menge an Kohlenstoffdioxid der Haut oder einer Wunde zur Verfügung gestellt wird. Darüber hinaus soll eine Vorrichtung bereitgestellt werden, die über einen längeren Zeitraum eine definierte Menge Kohlenstoffdioxid zur Behandlung von trophischen Störungen bereitstellt.

Gelöst wird die Aufgabe durch eine Vorrichtung gemäß des Anspruchs 1. Die Vorrichtung umfasst demnach eine flüssigkeitsdichte Folie, die im anwendungsgerechten Zustand der Vorrichtung als äußere Lage angeordnet ist, Mittel zur Erzeugung von Kohlendioxid, eine wässrige Phase zur Lösung des Kohlenstoffdioxids, ein Depot für die wässrige Phase und eine auf der Haut oder der Wunde zu liegen kommende erste Abstandsschicht, die das Depot von der Haut oder der Wunde beabstandet, wobei die erste Abstandsschicht ein Gewebe, Gestrick oder Gewirk aus einem hydrophoben Polymermaterial ist.

Mit dieser Vorrichtung wird eine Vorrichtung zur Verfügung gestellt, die zwei Behandlungsmethoden gleichzeitig unterstützt. Mit dieser Vorrichtung kann durch die Verwendung einer Lösung, in der Kohlenstoffdioxid gelöst vorliegt, eine Behandlung mittels Kohlenstoffdioxid mit der Methode der feuchten Wundbehandlung kombiniert werden.

Durch die erste Abstandsschicht wird gewährleistet, dass einerseits das Depot nicht direkt mit der zu behandelnden Haut oder der Wunde in Berührung gebracht wird und andererseits eine Zirkulation der wässrigen Lösung auf der Haut oder der Wunde und zwischen dem Depot und der Haut oder der Wunde gewährleistet ist. Unter einer Abstandsschicht wird im Rahmen dieser Erfindung eine Schicht verstanden, die ober- oder unterhalb des Depots angeordnet ist und keine absorbierenden Eigenschaften aufweist, dass heißt diese Abstandsschicht absorbiert im wesentlichen keine wässrigen Flüssigkeiten, insbesondere besteht die Abstandsschicht aus einem wässrige Flüssigkeiten nicht absorbierenden Material. Dadurch wird erreicht, dass die Haut oder die Wunde über den gesamten Zeitraum der Benutzung der Vorrichtung mit einer ausreichenden Menge an Kohlenstoffdioxid versorgt wird. Zudem wird durch die Abstandsschicht ein Konzentrationsgradient innerhalb der Wundauflage aufgebaut. Dieser Konzentrationsgradient kommt dadurch zustande, dass Kohlenstoffdioxid von der Haut oder der Wunde aufgenommen und andererseits aus dem Depot entnommen wird. Je größer der Abstand zwischen der Haut oder der Wunde und dem Depot ist, umso höher die Konvektion an gelöstem Kohlenstoffdioxid in der Wundauflage. Diese Konvektion gewährleistet zusätzlich, dass an allen zu behandelnden Stellen der Haut oder der Wunde eine gleichmäßige Konzentration an Kohlenstoffdioxid in der wässrigen Lösung vorliegt.

Gemäß der vorliegenden Erfindung weist die Abstandshalterschicht hydrophobe Eigenschaften auf. Hierdurch kann im Bereich der Auflagefläche der Abstandsschicht auf der Haut oder der Wunde die Gefahr der Schädigung der zu behandelnden Haut oder Wunde wesentlich reduziert werden. So wird zum Beispiel ein Verkleben der Vorrichtung mit der Wunde verhindert, falls die Vorrichtung über den Zeitraum mehrerer Tage zur Anwendung gebracht wird.

Vorzugsweise enthält die wässrige Phase der erfindungsgemäßen Vorrichtung mindestens 3 ml, insbesondere mindestens 5 ml, und weiter insbesondere mindestens 8 ml einer wässrigen Flüssigkeit. Dies stellt sicher, dass zum einen ausreichend Kohlendioxid in Lösung gehen kann und zum anderen die Vorrichtung den Anforderungen zur feuchten Wundbehandlung gerecht werden kann.

Hervorzuheben ist, dass es sich bei einer anspruchsgemäßen Erfindung auch um ein System handeln kann, dass die Bestandteile der Vorrichtung des ersten Anspruchs umfasst, wobei jeder Bestandteil oder jede Gruppe von Bestandteilen der Vorrichtung vor Gebrauch separat vorliegt und erst bei Gebrauch die Bestandteile miteinander kombiniert werden. Als Bestandteile sind hier insbesondere die flüssigkeitsdichte Folie, die wässrige Phase und eine Konstruktion umfassend ein Mittel zur Erzeugung von Kohlendioxid, ein Depot und eine Abstandsschicht gemeint. Insbesondere oder alternativ kann auch ein System vorgesehen sein, in dem lediglich eine flüssigkeitsdichte Folie vor Gebrauch getrennt von den übrigen Bestandteilen vorliegt und erst bei Gebrauch die Bestandteile miteinander kombiniert werden.

Als vorteilhaft hat sich erwiesen, dass die Vorrichtung eine zusätzliche zweite Abstandsschicht zwischen dem Depot und der äußeren Lage aufweist. In einer weiteren vorteilhaften Gestaltung der vorliegenden Erfindung ist vorgesehen, dass die zweite Abstandsschicht aus einem Gestrick, Gewirk oder Gewebe aus einem hydrophoben Polymermaterial besteht. Diese Art der Abstandsschicht gewährleistet besonders gut eine Zirkulation der wässrigen Lösung um/ auf die/ der zu behandelnde(n) Haut oder Wunde. In einer weiteren besonders bevorzugten Ausführung umfasst das Gestrick, Gewirk oder Gewebe ein hydrophobes Polyethylen-, Polypropylen-, Polyester-oder Viskose-Polymermaterial. Durch die Ausgestaltung als Gestrick, Gewirk oder Gewebe kann die Abstandsschicht in einer oder mehreren Richtungen gedehnt oder verformt werden, ohne dass sie sich von selbst wieder zusammenzieht oder ausrichtet. Als besonders vorteilhaft haben sich Gestricke oder Gewirke ausgezeichnet, die eine Dicke von mindestens 0,5 mm aufweisen. Insbesondere haben sich dabei Gestricke oder Gewirke ausgezeichnet, die eine Dicke von 0,5 bis 5,0 mm aufweisen. Die Oberfläche einer derartigen Abstandsschicht gleicht sich zudem passgenau an die Oberfläche der zu behandelnden Haut oder Wunde an.

Als weiterhin vorteilhaft hat sich herausgestellt, dass die Vorrichtung mindestens eine Verteilerschicht $V_n$ zwi-

schen der ersten Abstandschicht und dem Depot umfasst, wobei n = 1 bis 5 bedeutet und $V_n$ mit n = 1 die der Haut oder der Wunde am nächsten liegende Schicht und $V_n$ mit n = 5 die der Haut oder der Wunde am weitesten entfernte Schicht bedeutet. Dies gewährleistet eine besonders gleichmäßige Verteilung der Flüssigkeit oberhalb der Haut oder einer Wunde. Insbesondere hat sich herausgestellt, dass eine Verteilerschicht $V_n$ mit n = 1 ausgebildet als ein Tissue, Nonwoven, Gewebe, Gestrick und/oder Gewirk besonders leistungsfähig ist.

Zusätzlich oder alternativ zu der Verteilerschicht $V_n$ kann die Vorrichtung mindestens eine Verteilerschicht $V_m$ zwischen dem Depot und der zweiten Abstandsschicht umfassen, wobei m = 1 bis 5 bedeutet und $V_m$ mit m = 1 die der Haut oder der Wunde am entferntesten liegende Schicht und $V_m$ mit m = 5 die der Haut oder der Wunde am nächsten liegende Schicht bedeutet. Dabei kann die Vorrichtung, die mindestens eine Verteilerschicht aufweist, eine Verteilerschicht $V_n$ mit n = 1 oder 2 und/ oder eine Verteilerschicht $V_m$ mit m = 1 oder 2 aus einem Nonwoven aus einem hydrophilen Fasermaterial aufweisen. Hierbei kann als hydrophiles Fasermaterial insbesondere wasserunlösliche Fasern aus Cellulose, insbesondere weitgehend delignifizierte technische Zellstofffasern, insbesondere Holzzellstofffasern, insbesondere einer Faserlänge von < 5mm Verwendung finden. Das Fasermaterial kann auch hydrophiles Fasermaterial aus regenerierter Cellulose, Carboxymethylcellulose, Carboxyethylcellulose, Hydroxymethylcellulose oder Hydroxyethylcellulose enthalten.

In einer besonders bevorzugten Ausführungsform weist die Vorrichtung einen symmetrischen Aufbau auf, d.h. die Anzahl der Schichten mit derselben Funktion ist auf beiden Seiten des Depots gleich. Bei dieser Betrachtungsweise ist die als äußere Lage angeordnete flüssigkeitsdichte Folie nicht einbezogen.

Die im anwendungsgerechten Zustand der Vorrichtung als äußere Lage angeordnete flüssigkeitsdichte Folie, kann aus einer Vielzahl an Polymermaterialien gefertigt sein. Als besonders geeignet haben sich flüssigkeitsdichte und wasserdampfdurchlässige Folien gezeigt. Bei der Auswahl der Materialien, aus dem eine als äußere Lage angeordnete flüssigkeitsdichte und wasserdampfdurchlässige Folie besteht, ist besonders Augenmerk darauf zu richten, dass die Materialien wasserdampfdurchlässig sind und keinen oder wenn nur eine geringe Menge an Kohlenstoffdioxid durch die Folie durchtreten lassen. Als besonders geeignet haben sich hier flüssigkeitsdichte und wasserdampfdurchlässige Folien aus Polyester, Copolyester, Polyurethan, Polyetherpolyurethan, Polyesterpolyurethan, Polypropylen, Polyethylen oder Polyamid gezeigt. Besonders vorteilhaft haben sich unilaminare Folien mit einer Dicke von 20 bis 150 $\mu$m herausgestellt. Darüber hinaus haben sich Filme, die eine Wasserdampfdurchlässigkeit von mindestens 1000 g/ m$^2$/ 24 Std. und vorzugsweise von mindestens 1200 g/ m$^2$/ 24 Std. (gemessen nach DIN EN 13726) als besonders hautfreundlich herausgestellt. In besonders bevorzugten Ausführungsformen weisen diese Filme einen feuchtigkeitsdichten, klebenden Randabschnitt auf. Dieser Randabschnitt gewährleistet, dass die Vorrichtung an seinem bestimmungsgemäßen Ort appliziert und fixiert werden kann. Darüber hinaus ist sichergestellt, dass keine Flüssigkeit oder $CO_2$ zwischen der Folie und der die zu behandelnden Fläche umgebenden Haut austreten kann. Hierbei können alle bekannten feuchtigkeitsresistenten Klebstoffe Anwendung finden. Als besonders bevorzugt sind solche Klebstoffe zu betrachten, die in einem dünnen Auftrag von 20 bis 35 g/ m$^2$ zusammen mit dem Film eine Wasserdampfdurchlässigkeit von mindestens 800 g/ m$^2$/ 24 Std. und vorzugsweise von mindestens 1000 g/ m$^2$/ 24 Std. (gemessen nach DIN EN 13726) aufweisen.

In einer alternativen Ausgestaltung der Erfindung kann die äußere Lage mindestens ein Ventil umfassen. Dieses Ventil kann als Gasauslassventil ausgestaltet sein. So kann bei einem zu großem Überschuss an Kohlenstoffdioxid und dem damit einhergehenden Überdruck eine Teilmenge des Kohlenstoffdioxids aus der Vorrichtung abgelassen werden. Vorzugsweise kann es sich bei dem Ventil um ein Überdruckventil handeln, das selbstständig bei einem voreingestellten Druck öffnet. Alternativ oder zusätzlich kann in der äußeren Lage auch ein Einlassventil für eine wässrige Lösung vorgesehen sein. Dieses Ventil ist in einer besonderen Ausführungsform nur in eine Richtung, nämlich von außen in das Innere der Vorrichtung durchlässig. Somit kann eine Vorrichtung bereitgestellt werden, die erst nach dem applizieren auf der zu behandelnden Haut oder Wunde mit einer wässrigen Lösung beaufschlagt wird.

In einer besonders vorteilhaften Ausbildung der Erfindung kann die Vorrichtung eine wässrige Lösung umfassen, deren Gehalt an Kohlenstoffdioxid mehr als 1 g $CO_2$/l insbesondere mehr als 2g $CO_2$/ l bei 25 °C und Normaldruck beträgt. In einer Weiterführung der vorliegenden Erfindung weist die Vorrichtung in der wässrigen Lösung neben dem gelösten Kohlenstoffdioxid weitere die Haut stimulierende oder die Wundheilung fördernde Substanzen auf. Hierbei haben sich in besonderer Weise Vitamine oder Provitamine und deren Derivate als besonders wirkungsvoll bei der Heilung von Wunden gezeigt, wobei insbesondere Vitamin C, E und A hervorzuheben sind. Insbesondere umfasst die Vorrichtung in der wässrigen Lösung gelöste Elektrolyte. Als Elektolyte können hierbei Chloride, Iodide, Sulfate, Hydrogensulfate, Karbonate, Hydrogenkarbonate, Phosphate, Dihydrogenphosphate oder Hydrogenphosphate der Alkali- und Erdalkalimetalle sowie von Kupfer, Silber oder Zink Verwendung finden. Insbesondere kann als Elektrolyt Natrium-, Kalium-, Kalzium-, Zink- und/ oder Silberchlorid Verwendet werden.

In einer Weiterbildung der vorliegenden Erfindung wird in der Vorrichtung als Depot ein flüssigkeitsabsorbierendes Material verwendet. Dieses flüssigkeitsabsorbierende Material umfasst dabei vorzugsweise ein natürliches oder synthetisches superabsorbierendes Polymer. Unter einem superabsorbierendem Polymer wird im Rahmen der vorliegenden Erfindung ein Polymer verstanden, dass in der Lage ist, mindestens das 15-fache seines Eigengewichtes an physiologischer Kochsalzlösung (0,9 %-ige NaCl) zu absorbieren. Insbesondere kann es sich bei dem synthetischen superabsorbierenden Material um ein vernetztes Polyacrylat und ganz besonders bevorzugt um ein vernetztes Natriumsalz eines Polyacrylates handeln. In einer besonders vorteilhaften Ausführung besteht das Depot aus einem synthetischen superabsorbierenden Polymer. In jedem Fall kann das superabsorbierende Polymer in Partikelform vorliegen. Dabei zeigt sich zudem superabsorbierendes Polymer als besonders bevorzugt, das eine Partikelgrößenverteilung aufweist, deren Anteil an Partikel zwischen 850 $\mu$m und 300 $\mu$m mindestens 55 %, insbesondere mindestens 65 % und ganz besonders mindestens 70 % beträgt (gemessen nach EDANA 420.2-02). Insbesondere sind auch superabsorbierende Polymere bevorzugt, deren Partikelanteil an Partikeln kleiner als 850 $\mu$m mindestens 85 %, besonders mindestens 90 % und ganz besonders mindestens 95 % bezogen auf alle Partikel beträgt (gemessen nach EDANA 420.2-02).

Alternativ kann die Vorrichtung auch ein Depot umfassen, das ein synthetisches superabsorbierendes Polymer wie z.B. das bereits erwähnte vernetzte Polyacrylat-Polymer oder ein Polymethacrylat-Polymer und ein hydrophiles Fasermaterial aufweist. Hierbei kann als hydrophiles Fasermaterial insbesondere wasserunlösliche Fasern aus Cellulose, insbesondere weitgehend delignifizierte technische Zellstofffasern, insbesondere Holzzellstofffasern, insbesondere einer Faserlänge von < 5mm Verwendung finden. Das Fasermaterial kann auch hydrophiles Fasermaterial aus regenerierter Cellulose, Carboxymethylcellulose, Carboxyethylcellulose, Hydoxymethylcellulose oder Hydroxyethylcellulose enthalten. Es kann auch vorgesehen sein eine Fasermischung aus Cellulose-, regenerierter Cellulose, Carboxymethylcellulose-, Carboxyethylcellulose-, Hydoxymethylcellulose- oder Hydroxyethylcellulose-Fasern und Fasern aus Polyethylen, Polypropylen oder Polyester vorzusehen. In einer ganz besonders bevorzugten Ausführungsform umfasst das Depot eine Mischung aus Cellulosefasern, Polypropylenfasern und partikelförmigem superabsorbierenden Polymer. In einer besonderen Ausgestaltung der Erfindung ist vorgesehen, dass ein System umfassend eine Vorrichtung der beschriebenen Art in einer Verpackung angeordnet ist. Insbesondere ist dabei vorgesehen, dass die Verpackung eine sterile Verpackung ist. In einer besonders bevorzugten Ausführung dieses Systems liegt in der Verpackung oder in der sterilen Verpackung jeder einzelne Bestandteil oder jede Gruppe von Bestandteilen jeweils separat in Unterverpackungen vor. Es kann auch vorgesehen sein, dass jede Unterverpackung eine sterile Unterverpackung ist.

In einer bevorzugten Weiterbildung der Erfindung ist das Kohlendioxid erzeugende Mittel innerhalb des Depots für die wässrige Phase angeordnet. In einer weiteren Ausgestaltung dieses Erfindungsgedankens kann vorgesehen sein, dass das Kohlendioxid erzeugende Mittel mindestens ein Kompartiment umfasst, in dem Kohlenstoffdioxid chemisch gebunden vorliegt z.B. in Form eines Salzes der Kohlensäure. Dieses gebundene Kohlendioxid kann in dem Depot und damit innerhalb der Wundauflage durch z.B. eine organische Säure freigesetzt und in der wässrigen Phase gelöst werden. Somit wird eine Vorrichtung bereitgestellt, die Kohlenstoffdioxid innerhalb der Vorrichtung erzeugen kann. Als besonders bevorzugt werden hier Karbonate angesehen, die aus der Gruppe der Alkalimetall-, Erdalkalimetallkarbonate oder der Alkalimetall-, Erdalkalimetallhydrogenkarbonate gewählt werden. Hinsichtlich der Auswahl der organischen Säuren sind Mono-, Di- oder Tricarbonsäuren, Mono-, Di- und Trihydroxymonocarbonsäuren, Mono-, Di- und Trihydroxydicarbonsären und Mono-, Diund Trihydroxytricarbonsäuren als besonders bevorzugt zu nennen. Ganz besonders bevorzugt wird eine Vorrichtung, in der Kohlendioxid mittels eines Alkalimetallkarbonat oder Alkalimetallhydrogenkarbonat und einer Säure ausgewählt aus der Gruppe Zitronensäure, Milchsäure oder Weinsäure gebildet werden kann.

In einer besonders bevorzugten Vorrichtung ist vorgesehen, dass das Depot ein Karbonat oder eine organische Säure und ein superabsorbierendes Material umfasst. Hierbei hat sich als zweckmäßig gezeigt, wenn das Karbonat von dem superabsorbierende Material vollständig umgeben ist. In einer Ausgestaltung kann dabei weiterhin vorgesehen sein, das Karbonat in Form eines Pressstückes innerhalb des superabsorbierenden Materials einzubetten. In einer alternativen Ausführungsform kann auch vorgesehen sein, dass das Karbonat und das superabsorbierenden Material in Partikelform vorliegen. Hierbei hat sich gezeigt, dass das entstehende Kohlenstoffdioxid besonders gut in Lösung geht.

Alternativ kann die Vorrichtung auch ein Depot umfassen, das ein synthetisches superabsorbierendes Polymer wie z.B. das bereits erwähnte vernetzte Polyacrylat-Polymer oder ein Polymethacrylat-Polymer, ein hydrophiles Fasermaterial und ein Karbonat aufweist. Weiterhin kann vorgesehen sein eine organische Säure in einem weiteren Kompartiment des Depots vorzusehen, das verschieden ist von dem Kompartiment in dem das Karbonat vorliegt. Vorteilhafter Weise liegen die beiden Komponenten Carbonat und organische Säure auch in einem Kompartiment vor, wobei mindestens eine Komponente oder die Mischung der beiden Komponenten durch eine aktivierbare Schutzhülle umgeben ist. Besonders geeignet sind bei dieser Ausgestaltung der Erfindung solche Karbonate und organische Säuren, die wasserlöslich sind wogegen die Schutzhülle wasserunlöslich ist. Insbesondere ist vorgesehen eine wasserunlösliche Schutzhülle vorzusehen, die aktivierbar ist und die bei der Aktivierung, z.B. durch Zufuhr einer Dosis Energie in Form von Wärmestrahlung, die wasserlöslichen Bestandteile freisetzt. Hierdurch werden die wasserlöslichen Bestandteile gelöst und die Freisetzung des Kohlenstoffdioxids in Gang gesetzt. Mit dieser Ausgestaltung der Erfindung liegt eine Vorrichtung vor, mit der Kohlenstoffdioxid ständig nachgeliefert wird. Dies ist insbesondere der Fall, wenn die Schutzhüllen der einzelnen Kompartimente unterschiedlich gestaltet sind, so dass durch Beaufschlagung der Vorrichtung mit zeitlich versetzten Dosen oder Impulsen an Energie, wie z.B. Wärme, eine gezielte Freisetzung an Kohlenstoffdioxid erreicht werden kann. Als besonders bevorzugte Vorrichtung ist eine Vorrichtung anzusehen, in der in dem Depot eine organische Säure und/oder das Karbonat als Partikel oder Kapsel vorliegen, die mit einer wasserunlöslichen Schutzschicht versehen sind.

Weiterhin kann die Vorrichtung bereits im gebrauchsfertigen Zustand vorliegen. Bei dieser Ausgestaltung liegt das superabsorbierende Polymer des Depots als gelartige Partikel vor. In dem Polymer ist die wässrige Lösung gespeichert. Bei dieser Ausgestaltung ist weiterhin vorgesehen, dass das Kohlenstoffdioxid vorerst chemisch gebunden als Karbonat vorliegt. Hierbei liegen das Karbonat und die organische Säure in einem Schutzüberzug vor. Als besonders bevorzugter Schutzüberzug haben sich Wachse erwiesen. Diese Wachse sind wasserunlöslich und haben einen Schmelzpunkt oberhalb der Körpertemperatur. Insbesondere sind solche Wachse bevorzugt, die einen Schmelzpunkt zwischen 40 °C und 100 °C aufweisen. Wie oben bereits beschrieben wird durch die Aktivierung der Schutzhülle, hier durch Zufuhr von Wärme, z.B. durch Infrarotstrahlung, die Schutzhülle aktiviert, d.h. zerstört, das geschützte Karbonat und die geschützte organische Säure freisetzt, d.h. dass diese in Lösung gebracht werden durch die umgebende wässerige Lösung, so dass in einer Reaktion der beiden in Lösung befindlichen Komponenten nun Kohlenstoffdioxid gebildet und ebenfalls in Lösung gebracht werden kann.

Hervorzuheben ist an dieser Stelle, dass die hier aufgeführten Merkmale der alternativen Ausgestaltungen der Erfindungen nicht auf die einzelnen Alternativen zu beschränken sind. Es ist vielmehr der Fall, dass die Kombination der Ausgestaltungen bzw. die Kombination der Einzelmerkmale der alternativen Formen ebenso zu einer erfindungsgemäßen Ausgestaltung zu zählen ist. Ebenso wenig soll die Erfindung durch die nachfolgende Beschreibung der Zeichnungen reduziert verstanden sein.

Nachstehend wird die Erfindung unter Bezugnahme auf die Zeichnung erläutert. In den Zeichnungen zeigt:

Figur 1: eine erste Ausgestaltung einer erfindungsgemäßen Vorrichtung, im Querschnitt

Figur 2: eine weitere Ausführungsform einer erfindungsgemäßen Vorrichtung, im Querschnitt

Figur 3: eine dritte Ausführungsform einer erfindungsgemäßen Vorrichtung, im Querschnitt

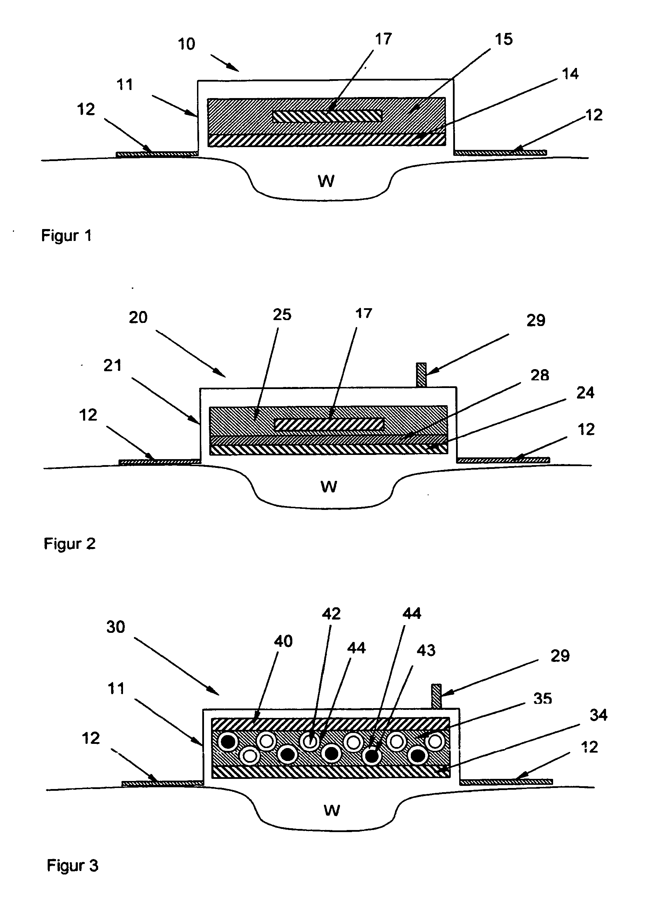

Mit der Figur 1 ist eine erste Ausgestaltung einer emndüngsgemäßen Vorrichtung nämlich eine Wundauflage (10) gezeigt, die oberhalb einer Wunde (W) liegt. Die Wundauflage weist als äußere Lage eine flüssigkeitsdichte Folie (11) auf, die an den Berührungsflächen mit der der Wunde umgebenden Haut über einen flüssigkeitsdichten, klebenden Randabschnitt (12) verbunden ist. Auf der der Wunde zugewandten Seite weist die Wundauflage eine erste Abstandsschicht (14) und darüber liegend ein Depot (15) für die wässrige Phase zur Lösung des Kohlenstoffdioxids auf. Innerhalb des Depots befindet sich das Kohlendioxid erzeugende Mittel in Form eines gepressten Formstückes (17), das in fester Form ein Alkalikarbonat und eine feste organische Säure umfasst. Dieses Formstück (17) gewährleistet, dass über einen Zeitraum von mindestens einem Tag die Konzentration an Kohlenstoffdioxid in der wässrigen Lösung mindestens 1 g/ l beträgt.

In Figur 2 ist ein alternativer Aufbau einer Wundauflage (20) gezeigt. Diese Wundauflage umfasst neben einer der Wunde (W) zugewandten ersten Abstandsschicht (24) zwischen dem Depot (25) und dieser Abstandsschicht (24) eine zusätzliche Verteilerschicht (28). Weiterhin ist in der äußere Lage (21) ein Auslassventil (29) für überschüssiges Kohlenstoffdioxid vorgesehen. Dieses Ventil ist als Einwegeventil derart gestaltet, dass bei einem Überdruck in der Wundauflage lediglich Gase vom inneren der Wundauflage in die äußere Umgebung austreten können. Gemäß einer hier nicht dargestellten Ausführungsform kann die Wundauflage ein Zu- bzw. Ablaufventil zur Entfernung von an Kohlenstoffdioxid verarmten wässrigen Lösungen bzw. Zugabe von frisch zubereiteten Kohlenstoffdioxid enthaltenden Lösungen aufweisen.

Mit Figur 3 ist eine weitere Wundauflage (30) mit einem alternativen Aufbau wiedergegeben. Diese Wundauflage weist eine erste Abstandsschicht (34) unterhalb des Depots (35) und eine zweite Abstandsschicht (40) oberhalb des Depots auf. Innerhalb des Depots (35) sind Partikel oder Kapseln vorgesehen, die aus Natriumkarbonat (42) oder Zitronensäure (43) bestehen. Diese Kapseln sind mit einer wasserlöslichen Schutzschicht (44) versehen. Mit diesem Aufbau einer Wundauflage bzw. durch diese Schutzschichten ist gewährleistet, dass die wässrige Lösung kontinuierlich mit Kohlenstoffdioxid angereichert wird.

Ausführungsbeispiel

Die Wundauflage weist als äußere Lage einen flüssigkeitsdichten Polyurethanfilm auf. Der Polyurethanfilm (rechteckige Grundform 10 x 15 cm, mit einer Dicke von 30 $\mu$m), ist vollflächig mit einem wasserresistenten Acrylat-Klebstoff (35 g/ m$^2$) beschichtet und weist zusammen mit dem Haftkleber eine Wasserdampfdurchlässigkeit von 1300 g/ m$^2$/ 24 Std. (gemessen nach DIN EN 13726) auf. Auf der der Wunde zugewandten Seite weist die Wundauflage eine erste Abstandsschicht, darüber liegend eine erste Verteilerschicht, darüber liegend ein Depot für die das Kohlenstoffdioxid umfassende wässrige Phase, darüber angeordnet eine zweite Verteilerschicht und darüber eine zweite Abstandsschicht auf. Die beiden Abstandsschichten sind entlang ihrer Ränder miteinander verbunden, so dass diese eine geschlossene Hülle für das Depot und für die zwei Verteilerschichten bildet. Hierbei ist die Abstandschicht jeweils aus einem hydrophoben Gestrick aus Polypropylen gefertigt, das im entspannten Zustand eine Dicke von 0,8 mm (gemessen bei einem Prüfdruck von 0,5 kPa) aufweist. Durch die vorzügliche Permeabilität der Abstandsschicht wird die Flüssigkeit sehr rasch durch die Abstandsschicht hindurch zur Wunde oder von der Wunde her in das Depot hinein abgeleitet. Dabei nimmt die Abstandsschicht kaum Feuchtigkeit auf. Die beiden Verteilerschichten, wovon jeweils eine auf einer Seite des Depots liegt, sind aus einem aus 100 % Cellulosefasern gefertigten Tissue mit einem Flächengewicht von 18 g/ m² gefertigt. Das Depot mit einem Flächengewicht von 340 g/ m² besteht aus einem luftgelegten 1:1-Gemisch aus Fasern und superabsorbierendem Natriumpolyacrylat (Favor® Z 3034, Fa. Degussa). Das Natriumpolyacrylat liegt in Partikelform vor und weist einen Partikelanteil von ca. 99 % auf, der eine Partikelgröße von kleiner 0,85 mm betragen. Ca. 80 % der Partikel weisen eine Größe von 0,30 - 0,85 mm auf (gemessen nach EDANA 420.2-02). Das Fasermaterial des Depots ist ein Gemisch aus 94 Gew.-% 100 %-igen hydrophilen Cellulose-Fasern und 6 Gew.-% 100%-igen Polypropylenfasern. In dem Depot ist weiter als Kohlendioxid erzeugendes Mittel eine gepresste Tablette bestehend aus Natriumhydrogenkarbonat und Zitronensäure in der Art eingebracht, dass diese Tablette vollständig von dem Gemisch aus superabsorbierendem Natriumpolyacrylat und Fasermaterial umgeben ist. Diese Tablette mit einem Gewicht von 7 g, in der Natriumhydrogenkarbonat und Zitronensäure in equimolaren Mengen vorliegen, gewährleistet, dass über einen Zeitraum von mindestens einer Stunde ständig Kohlenstoffdioxid in die wässrige Lösung nachgeliefert wird, so dass die Konzentration an Kohlenstoffdioxid über 4 Std. gemessen in der wässrigen Lösung mindestens 1 g/ l/ (bei 25°C und Normaldruck) beträgt. Die wässrige Lösung weist neben dem Kohlenstoffdioxid ein isotonisches Gemisch an Magnesiumchlorid, Kalziumchlorid und Kaliumchlorid auf.

Anwendung:

Eine wässrige Lösung, die ein isotonisches Gemisch an Magnesiumchlorid, Kalziumchlorid und Kaliumchlorid enthält, liegt vor Anwendung dieser Wundauflage in einer separaten 15 ml Ampulle vor. Die Ampulle ihrerseits und die steril verpackte Wundauflage mit einer Auflagefläche von 25 cm² liegen vor der Anwendung zusammen in einer gemeinsamen, sterilen Verpackung vor. Kurz vor der Anwendung wird die Wundauflage mit der gesamten Menge an wässriger Lösung gesättigt. Bei Kontakt mit Wasser entwickelt sich sofort im Inneren der Wundauflage Kohlenstoffdioxid, das durch die relativ große Oberfläche des umgebenden Materials gut in Lösung geht. Die Wundauflage wird sodann auf der zu behandelnden Stelle appliziert und mit Hilfe des ebenfalls in einer separaten Unterverpackung vorliegenden Polyurethanfilms fixiert.

## Patentansprüche

1. Vorrichtung (10, 20, 30) zur Behandlung von trophischen Störungen in einer Haut oder in einer Wunde mittels Kohlenstoffdioxid umfassend eine im anwendungsgerechten Zustand der Vorrichtung als äußere Lage angeordnete flüssigkeitsdichte Folie (11, 21), Mittel zur Erzeugung von Kohlendioxid (17), eine wässrige Phase zur Lösung des Kohlenstoffdioxids und ein Depot (15, 25, 35) für die wässrige Phase **dadurch gekennzeichnet, dass** eine auf der Haut oder der Wunde zu liegen kommende, erste Abstandsschicht (14, 24, 34) das Depot (15, 25, 35) von der Haut oder der Wunde beabstandet, wobei die erste Abstandsschicht ein Gewebe, Gestrick oder Gewirk aus einem hydrophoben Polymermaterial ist.

2. Vorrichtung (10, 20, 30) nach Anspruch 1 **dadurch gekennzeichnet, dass** die Vorrichtung eine zweite Abstandsschicht (40) zwischen dem Depot (15, 25, 35) und der äußeren Lage (11, 21) umfasst.

3. Vorrichtung (10, 20, 30) nach Anspruch 1 oder 2 **dadurch gekennzeichnet, dass** die zweite Abstandsschicht (40) ein Gewebe, Gestrick oder Gewirk aus einem hydrophoben Polymermaterial ist.

4. Vorrichtung (10, 20, 30) nach mindestens einem der vorgenannten Ansprüche **dadurch gekennzeichnet, dass** die Vorrichtung (10, 20, 30) mindestens eine erste Verteilerschicht $V_n$ (28) zwischen der ersten Abstandschicht (14, 24, 34) und dem Depot (15, 25, 35) umfasst, wobei n = 1 bis 5 bedeutet und $V_n$ mit n = 1 die der Haut oder der Wunde am nächsten liegende Schicht und $V_n$ mit n = 5 die der Haut oder der Wunde am weitesten entfernte Schicht bedeutet.

5. Vorrichtung (10, 20, 30) nach mindestens einem der vorhergehenden Ansprüche **dadurch gekennzeichnet, dass** die erste Verteilerschicht $V_n$ mit n = 1 ein Tissue, Nonwoven, Gewebe, Gestrick und/oder Gewirk ist.

6. Vorrichtung (10, 20, 30) nach mindestens einem der vorhergehenden Ansprüche **dadurch gekennzeichnet, dass**

die erste Verteilerschicht $V_n$ mit n = 1 oder 2 ein Nonwoven aus einem hydrophilen Fasermaterial umfasst.

7. Vorrichtung (10, 20, 30) nach mindestens einem der vorhergehenden Ansprüche **dadurch gekennzeichnet, dass** das Depot (15, 25, 35) ein flüssigkeitsabsorbierendes Material umfasst.

8. Vorrichtung (10, 20, 30) nach mindestens einem der vorhergehenden Ansprüche **dadurch gekennzeichnet, dass** das flüssigkeitsabsorbierende Material ein natürliches oder synthetisches superabsorbierendes Polymer umfasst.

9. Vorrichtung (10, 20, 30) nach mindestens einem der vorhergehenden Ansprüche **dadurch gekennzeichnet, dass** das flüssigkeitsabsorbierende Material ein synthetisches superabsorbierendes Polymer ist.

10. Vorrichtung (10, 20, 30) nach mindestens einem der vorhergehenden Ansprüche **dadurch gekennzeichnet, dass** die äußere Lage mindestens ein Ventil (29) umfasst.

11. Vorrichtung (10, 20, 30) nach mindestens einem der vorhergehenden Ansprüche **dadurch gekennzeichnet, dass** die äußere Lage ein Einlassventil für die wässrige Lösung umfasst.

12. Vorrichtung (10, 20, 30) nach mindestens einem der vorhergehenden Ansprüche **dadurch gekennzeichnet, dass** die äußere Lage ein Auslassventil für überschüssiges Gas umfasst.

13. Vorrichtung (10, 20, 30) nach mindestens einem der vorhergehenden Ansprüche **dadurch gekennzeichnet, dass** der Gehalt an Kohlenstoffdioxid in der wässrige Lösung mehr als 1 g $CO_2$ / und insbesondere mehr als 2 g $CO_2$ / l bei 25 °C und Normaldruck beträgt.

14. Vorrichtung (10, 20, 30) nach mindestens einem der vorhergehenden Ansprüche **dadurch gekennzeichnet, dass** die wässrige Lösung weiterhin mindestens ein Elektrolyt umfasst ausgewählt aus der Gruppe Natriumchlorid, Kalziumchlorid, Kaliumchlorid, Zinkchlorid und/ oder Silberchlorid.

15. Vorrichtung (10, 20, 30) nach mindestens einem der vorhergehenden Ansprüche **dadurch gekennzeichnet, dass** das Kohlendioxid mittels eines Alkalimetallkarbonat oder Alkalimetallhydrogenkarbonat und einer Säure ausgewählt aus der Gruppe Zitronensäure, Milchsäure oder Weinsäure gebildet wird.

16. Vorrichtung (20) nach mindestens einem der vorhergehenden Ansprüche **dadurch gekennzeichnet, dass** das Kohlendioxid erzeugende Mittel (17) innerhalb des Depots (25) für die wässrige Phase angeordnet ist.

17. Vorrichtung (20) nach Anspruch 16, **dadurch gekennzeichnet, dass** das Kohlendioxid erzeugende Mittel (17) mindestens ein Kompartiment umfasst, in dem Kohlenstoffdioxid in Form eines Salzes der Kohlensäure chemisch gebunden vorliegt.

**Claims**

1. Device (10, 20, 30) for treating trophic disorders in the skin or in a wound by means of carbon dioxide, comprising a liquid-impermeable film (11, 21), which is arranged as an outer sheet when the device is in the state in which it is intended to be used, means (17) for generating carbon dioxide, an aqueous phase for dissolving the carbon dioxide, and a reservoir (15, 25, 35) for the aqueous phase, **characterized in that** a first spacer layer (14, 24, 34), which comes to lie on the skin or the wound, spaces the reservoir (15, 25, 35) away from the skin or the wound, said first spacer layer being a woven fabric or knitted fabric composed of a hydrophobic polymer material.

2. Device (10, 20, 30) according to Claim 1, **characterized in that** the device comprises a second spacer layer (40) between the reservoir (15, 25, 35) and the outer sheet (11, 21).

3. Device (10, 20, 30) according to Claim 1 or 2, **characterized in that** the second spacer layer (40) is a woven fabric or knitted fabric composed of a hydrophobic polymer material.

4. Device (10, 20, 30) according to at least one of the aforementioned claims, **characterized in that** the device (10, 20, 30) comprises at least a first distributor layer $V_n$ (28) between the first spacer layer (14, 24, 34) and the reservoir (15, 25, 35), where n = 1 to 5, and $V_n$ with n = 1 means the layer lying nearest to the skin or to the wound, and $V_n$

with n = 5 means the layer farthest away from the skin or the wound.

5. Device (10, 20, 30) according to at least one of the preceding claims, **characterized in that** the first distributor layer $V_n$ with n = 1 is a tissue, a nonwoven fabric, a woven fabric or a knitted fabric.

6. Device (10, 20, 30) according to at least one of the preceding claims, **characterized in that** the first distributor layer $V_n$ with n = 1 or 2 comprises a nonwoven fabric composed of a hydrophilic fibre material.

7. Device (10, 20, 30) according to at least one of the preceding claims, **characterized in that** the reservoir (15, 25, 35) comprises a liquid-absorbing material.

8. Device (10, 20, 30) according to at least one of the preceding claims, **characterized in that** the liquid-absorbing material is a natural or synthetic superabsorbent polymer.

9. Device (10, 20, 30) according to at least one of the preceding claims, **characterized in that** the liquid-absorbing material is a synthetic superabsorbent polymer.

10. Device (10, 20, 30) according to at least one of the preceding claims, **characterized in that** the outer sheet comprises at least one valve (29).

11. Device (10, 20, 30) according to at least one of the preceding claims, **characterized in that** the outer sheet comprises an inlet valve for the aqueous solution.

12. Device (10, 20, 30) according to at least one of the preceding claims, **characterized in that** the outer sheet comprises an outlet valve for excess gas.

13. Device (10, 20, 30) according to at least one of the preceding claims, **characterized in that** the content of carbon dioxide in the aqueous solution is more than 1 g $CO_2$/l, and in particular more than 2 g $CO_2$/l at 25°C and normal pressure.

14. Device (10, 20, 30) according to at least one of the preceding claims, **characterized in that** the aqueous solution further comprises at least one electrolyte chosen from the group including sodium chloride, calcium chloride, potassium chloride, zinc chloride and/or silver chloride.

15. Device (10, 20, 30) according to at least one of the preceding claims, **characterized in that** the carbon dioxide is formed by means of an alkali metal carbonate or alkali metal hydrogen carbonate and an acid chosen from the group including citric acid, lactic acid and tartaric acid.

16. Device (20) according to at least one of the preceding claims, **characterized in that** the means (17) generating carbon dioxide is arranged inside the reservoir (25) for the aqueous phase.

17. Device (20) according to Claim 16, **characterized in that** the means (17) generating carbon dioxide comprises at least one compartment in which carbon dioxide is chemically bound in the form of a salt of carbonic acid.

**Revendications**

1. Dispositif (10, 20, 30) pour traiter par dioxyde de carbone des perturbations trophiques de la peau ou une blessure, comprenant une feuille (11, 21) étanche aux liquides et disposée comme couche extérieure lorsque le dispositif est prêt à l'emploi,
des moyens de production de dioxyde de carbone (17),
une phase aqueuse de dissolution du dioxyde de carbone et
une réserve (15, 25, 35) de phase aqueuse, **caractérisé en ce que** une première couche d'écartement (14, 24, 34) qui vient se placer sur la peau ou la blessure maintient la réserve (15, 25, 35) à distance de la peau ou de la blessure et
**en ce que** la première couche d'écartement est un tissu ou un tricot en matériau polymère hydrophobe.

2. Dispositif (10, 20, 30) selon la revendication 1, **caractérisé en ce que** le dispositif comprend une deuxième couche d'écartement (40) entre la réserve (15, 25, 35) et la couche extérieure (11, 21).

3. Dispositif (10, 20, 30) selon la revendication 1 ou 2, **caractérisé en ce que** la deuxième couche d'écartement (40) est un tissu ou un tricot en un matériau polymère hydrophobe.

4. Dispositif (10, 20, 30) selon au moins l'une des revendications précédentes, **caractérisé en ce que** le dispositif (10, 20, 30) comprend au moins une première couche de répartition $V_n$ (28) entre la première couche d'écartement (14, 24, 34) et la réserve (15, 25, 35), **en ce que** n = 1 à 5 et **en ce que** $V_n$ représente la couche située le plus près de la peau ou de la blessure lorsque n = 1 et la couche la plus éloignée de la peau ou de la blessure lorsque n = 5.

5. Dispositif (10, 20, 30) selon au moins l'une des revendications précédentes, **caractérisé en ce que** lorsque n = 1, la première couche de répartition $V_n$ est une toile, un non tissé, un tissu et/ou un tricot.

6. Dispositif (10, 20, 30) selon au moins l'une des revendications précédentes, **caractérisé en ce que** lorsque n = 1 ou 2, $V_n$ comprend un non tissé en un matériau fibreux hydrophile.

7. Dispositif (10, 20, 30) selon au moins l'une des revendications précédentes, **caractérisé en ce que** la réserve (15, 25, 35) contient un matériau absorbant les liquides.

8. Dispositif (10, 20, 30) selon au moins l'une des revendications précédentes, **caractérisé en ce que** le matériau absorbant les liquides contient un polymère superabsorbant, naturel ou synthétique.

9. Dispositif (10, 20, 30) selon au moins l'une des revendications précédentes, **caractérisé en ce que** le matériau absorbant les liquides est un polymère superabsorbant synthétique.

10. Dispositif (10, 20, 30) selon au moins l'une des revendications précédentes, **caractérisé en ce que** la couche extérieure contient au moins une soupape (29).

11. Dispositif (10, 20, 30) selon au moins l'une des revendications précédentes, **caractérisé en ce que** la couche extérieure contient une soupape d'admission d'une solution aqueuse.

12. Dispositif (10, 20, 30) selon au moins l'une des revendications précédentes, **caractérisé en ce que** la couche extérieure comprend une soupape d'échappement pour le gaz en excès.

13. Dispositif (10, 20, 30) selon au moins l'une des revendications précédentes, **caractérisé en ce que** la teneur en dioxyde de carbone dans la solution aqueuse est supérieure à 1 g $CO_2$/l et en particulier supérieure à 2 g $CO_2$/l à 25°C et sous pression normale.

14. Dispositif (10, 20, 30) selon au moins l'une des revendications précédentes, **caractérisé en ce que** la solution aqueuse comprend en outre au moins un électrolyte sélectionné dans l'ensemble constitué du chlorure de sodium, du chlorure de calcium, du chlorure de potassium, du chlorure de zinc et/ou du chlorure d'argent.

15. Dispositif (10, 20, 30) selon au moins l'une des revendications précédentes, **caractérisé en ce que** le dioxyde de carbone est formé au moyen d'un carbonate de métal alcalin ou d'un hydrogénocarbonate de métal alcalin et d'un acide sélectionné dans l'ensemble constitué de l'acide citrique, de l'acide lactique et de l'acide acétique.

16. Dispositif (20) selon au moins l'une des revendications précédentes, **caractérisé en ce que** le moyen (17) qui produit le dioxyde de carbone est disposé à l'intérieur de la réserve (25) de phase aqueuse.

17. Dispositif (20) selon la revendication 16, **caractérisé en ce que** le moyen (17) qui produit le dioxyde de carbone comprend au moins un compartiment dans lequel le dioxyde de carbone est lié chimiquement sous la forme d'un sel d'acide carbonique.

Figur 1

Figur 2

Figur 3

**EP 1 688 109 B1**

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- WO 01005445 A1
- US 20040260253 A1